# EUROPEAN PATENT APPLICATION

(11) **EP 1 813 253 A1**
(43) Date of publication of application: **01.08.2007**
(21) Application number: 07250260.2
(22) Date of filing: 23.01.2007
(51) Int. Cl.: A61K 8/04, A61K 8/11, A61Q 1/02, A61Q 1/04, A61Q 1/10, A61Q 5/02, A61Q 5/06, A61Q 5/12, A61Q 17/04

(54) **Cosmetic compositions comprising multiphasic particles**

(30) Priority: 23.01.2006 US 761180 P
(71) Applicant: L'Oréal, 75008 Paris (FR)
(72) Inventor: Quadir, Murat, New Jersey 07076 (US); Mathonneau, Estelle, 75010 Paris (FR)
(74) Representative: Tanty, François

(57) **Abstract**

The present invention relates to multiphasic particles (MPPs), their design, composition, and use. The present invention also relates to products, and in particular, cosmetics, personal care products, and topical or dermatological products, which may be produced using MPPs.

## Description

This application is based on and claims the benefit of U.S. Provisional Application Serial No. 60/761,180, entitled COSMETIC COMPOSITIONS COMPRISING MULTIPHASIC PARTICLES, filed January 23, 2006, the entire disclosure of which is incorporated by reference herein.

### BACKGROUND OF THE INVENTION

The use of particulate in various cosmetic, personal care and topical or dermatological products is well-known. Particulate materials in the form of pigments are often found in colored cosmetics and polymeric adhesive particles have been used in hair care products, such as hair styling products. *See* U.S. Patent No. 6,548,051 and U.S. Patent Application Publication No. 2002/0059941. Particulates have been used as filler and for many other purposes in cosmetics.

Yet the use of particulate materials can present problems in various formulations. It may be desired or necessary to impart more than one property through the use of solid fillers or particulate, and yet, to obtain sufficiently the benefits of each, one may need to add an amount of particulate which is, itself, problematic. It may be also difficult to mitigate or tailor the degree of certain properties imparted by a particulate. In other instances, providing accurate mixtures of particulate where particles are supposed to stay in close proximity to one another in a given format can be extremely difficult. One particle may be hydrophilic while the other particle is hydrophobic and while it is desirable to keep such particles in generally close proximity, their natural tendencies for one phase or another of an ultimate product may actually enhance the chance of, for example, phase separation or at least promote the separation of these disparate particles. Designing particles which would not suffer from these disadvantages could provide greater flexibility and enhance properties in cosmetics products, personal care products, and topical or dermatological products.

### SUMMARY OF THE INVENTION

The invention relates to a composition containing:
(a) a cosmetically acceptable carrier;
(b) multiphasic particles having a surface and at least two homogenous phases which provide homogenous areas at said surface, each area imparting a different property or function; and
(c) optionally, at least one cosmetic additive for imparting an additional cosmetic effect onto the keratinous substrate.
In addition, the invention relates to a process for imparting a visual and cosmetic effect onto a keratinous substrate involving:
(a) providing a cosmetic composition comprising:
   (i) a cosmetically acceptable carrier;
   (ii) multiphasic particles having a surface and at least two homogenous phases which provide homogenous areas at said surface, each area imparting a different property or function; and
   (iii) optionally, at least one cosmetic additive for imparting an additional cosmetic effect onto a keratinous substrate; and
(b) contacting the keratinous substrate with the cosmetic composition to form a treated keratinous substrate.

The MPPs in accordance with the present invention have an external area or external region (collectively "phase") of a first material having a first set of properties whose surface area is 90% or less and at least a second phase of a second material having a second set of properties distinct from the first set of properties whose surface area is 10% or more.

The MPPs of the present invention include a plurality of phases, each of which imparts a different predetermined property or function to products in accordance with the present invention. In different embodiments, at least one of the phases has a property selected from the group consisting of adhesiveness, hydrophobicity, hydrophillicity, positive and/or negative charge, texture, optical properties, and porosity.

The present invention relates to topical or dermatological products, cosmetics and personal care products (again collectively "products") directed for application to the skin, hair or nails, which comprise MPPs.

The present invention also relates to hair cleaning, conditioning, nourishing, and coloring products, such as shampoos, conditioners, leave in conditioners, permanent and semi-permanent dyes, which comprise MPPs. In another embodiment, the present invention relates to hair styling products such as gel, mousse, lotion, and spray, which comprise MPPs.

### BRIEF DESCRIPTION OF THE DRAWINGS

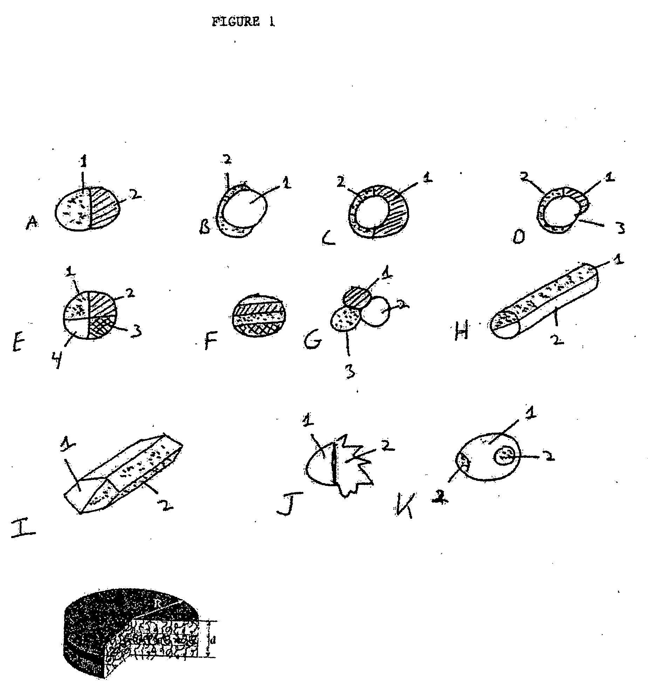

Figure 1 illustrates certain shapes and arrangements of phases in multiphase particles in accordance with certain embodiments of the invention.

### DETAILED DESCRIPTION

The present invention relates to multiphase particles ("MPPs"), their design, composition, and use. The present invention also relates to products, and in particular, cosmetics, personal care products, and topical or dermatological products, which may be produced using MPPs. The term "particle" as used herein in connection with MPPs refers to a solid or filled or semisolid entity, which exists, preferably, in a finely divided state. This can include powders, beads, chips, pellets, as well as grains, granulates, and agglomerates. It should be understood, however, that particles in accordance with the present invention as used in connection with MPPs can also embrace other structures including cylindrical particles of extended lengths and even strands, filaments, or fibers. These particles may be spherical, regularly shaped, irregularly shaped, shaped as a rod or have a polygon cross-section (triangular, trapezoid, octagonal, etc.).

Figure 1 illustrates some representative multiphasic particles in accordance with various aspects of the present invention. Particle A is made of two hemispheres each of a different material, 1 and 2 respectively. Particles B-D show particles in cross-section. Particle B includes a coating 2 over a portion of the surface area of core 1. The coating 2 is one phase and the core 1 including the exposed surface is the second phase. Particle C includes a core completely surrounded by a first coating 1 and a second coating 2. The coatings in this case are the two phases. Particle D is like particle C, except the two coatings do not completely cover the surface area of the core 3 and thus this particle has three phases, with the core 3 and its exposed surface being the third phase. Particle E includes 4 phases 1, 2, 3 and 4 and particle F has the same 4 phases in a different arrangement. Particle G is an irregular particle made using three phases, 1, 2 and 3 and particle H is a particle with a circular cross-section but a rod or cylindrical shape made of phases 1 and 2. Particle I is a biphasic particle in the shape of a rod with a hexagonal cross-section and particle J is a cross-sectional view of a particle of a first phase 1 with a smooth surface and a second phase 2 with a textured surface. Particle K is a core 1 of a first material having a discontinuous pattern of a second phase in various spots on it surface with the spots forming the second phase. Particle L is a multiphasic particle comprising at least three layers which form a multi-layered sheet.

"Multiphase" or "multiphasic" as used in connection with MPPs refers to particles having at least two or more regions or areas (collectively called "phases") at the surface of the particles wherein at least two phases are physically, structurally, or functionally discrete with respect to any other phases of that same particle. "Structure" is meant to mean the physical aspects of each phase, including, for example and without limitation, its depth, size, shape, topography or texture. "Function" is meant to describe the characteristics of a phase, such as, for example and without limitation, adhesiveness, optical properties and the like as described herein, and preferably to describe the characteristic imparted to the products contemplated herein. MPPs are constituted of different phases and these phases are either chemically or covalently bonded together or bonded via a topologically entrapped network or IPN (interpenetration network). The latter can be exemplified by a polymer comprising two (or more networks) which are at least partially interlaced on a molecular scale but not covalently bonded to each other, but which cannot be separated unless chemical bonds are broken. In addition, MPPs could include a first network entrapped in a second network. A phase is homogenous in its composition and its properties. The multiphasic particle or system can be accomplished using any number of shapes and structures. For example, an MPP can include a core particle, which is coated with two or more discrete materials on different portions of the surface of the particle. If the particle is completely covered by both as, for example, half of the surface coated with one and the other half coated with the other, the particle can be called biphasic. But, if the coating is incomplete and some of the surface area of the underlying particle is exposed, the particle is triphasic.

In a similar vein, a single particle could be coated on half, or some other proportion of its surface area, with a material having different properties, structure, or function. MPPs can also be envisioned by taking two or more like sized or two or more different sized particles, cutting a portion or section from each particle and chemically bonding the one section from each of the originally different particles together. One phase of the MPPs in accordance with the present invention has a surface area of at least about 10% of the particle.

These are but illustrations. Moreover, while the above examples generally describe particles comprising equal percentages of surface area for each component material, that need not be the case. A bead of one material could be coated with a very small dot, in only one specific spot, of a second material or could be overcoated over 90% of its surface area with a second material.

The MPPs in accordance with the present invention need not be solid or filled. They can be hollow and any particular phase or portion thereof may be porous, striated, regular or irregular. Indeed, an MPP in accordance with the present invention could be a bead wherein one portion or one surface is roughened or imprinted or otherwise formed with grooves or channels or even a grid while the other portion of the same bead is completely smooth.

Preferably, however, MPPs in accordance with the present invention are designed, and the materials used for their construction are selected, to provide MPPs having at least two homogenous phases having different chemical structures (or one of each), structures which lead to properties giving final particles which are useful in cosmetics, personal care products, and/or topical or dermatological products. These are collectively referred to herein as "products." Cosmetics, personal care products, and topical or dermatological products include, for example, products that are typically applied to accentuate, enhance, hide, color or otherwise change the appearance of a feature of the hair, skin or nails, such as hair coloring products, hair permanent, semipermanent, and temporary products, permanent waving products hair relaxers, products that clean, deodorize, moisturize, condition or change the skin or hair, prevent sweat and/or odor, add moisture and or conditioning and provide other essential grooming features, products that treat or prevent a condition of the skin or hair, sunscreens and hair growth promoters.

Preferably, particles of the invention have at least one hydrophilic phase. At least one other phase in such particles can be a hydrophilic or hydrophobic phase. Another preferred particle of the invention has at least one adhesive phase. The particles of the invention can also consist only of hydrophobic phases. Phases can be organic or inorganic. Preferably all phases are organic.

The MPPs in accordance with the present invention can be used in any such product in place of, or in addition to, any particles known for use in connection with same. They may be added to replace the functions provided by traditional monophasic particles, to provide additional benefits, or to tailor or mitigate the benefits or properties of existing particles in like products. They can also replace edible compounds. For instance, MPPs in accordance with certain aspects of the invention include two or more homogenous phases capable of providing, without limitation, a tacky or adhesive area, a nontacky or nonadhesive area, a hydrophilic area, a hydrophobic area, a positive charge, a negative charge (zwitterionic), a positive and negative charge, a porous area, a nonporous area, a rigid area, a flexible area, an area of bright finish, an area of matt finish, a transparent area, an opaque area, a colored area, a clear area, a diffractive area, a reflective area, an absorptive area, an antibiotic area, a UV-absorbing area, a UV-blocking area, a dense area, a smooth area, an irregular area, a textured area, a patterned area, a conductive area, a nonconductive area, a magnetic area, a metallic area, a reactive area, a nonreactive area, an area of generally good affinity for keratinaceous material and/or one that has no similar affinity or any contradiction of these. One phase of an MPP in accordance with the present invention could be extremely slippery and could act as a glidant. One area could have high lubricity. One area could be absorbent, allowing that phase to absorb fragrance, emollients, humectants, solvents and the like, while another area can be inert and incapable of any absorption. Similarly, one region could be hollow and contain a fragrance, an emollient, a humectant, a moisturizer, or could be impregnated therewith such that upon certain stimuli, or certain activity, the materials contained or impregnated therein are released.

For example, MPPs in accordance with the present invention can comprise: an anionic area and a cationic area; a hydrophilic area and a hydrophobic area; a tacky area and a non-tacky area; a shiny area and a matte area; a smooth area and a textured area; a colored area and a differently colored area; a colored area and an uncolored area; a rigid area and a soft area; a conductive area and a non-conductive area; a magnetic area and a non-magnetic area; a reflective area and a UV filter area; an adhesive to keratinous substrate area and a UV filter area; an adhesive to keratinous substrate area and a smooth area; an adhesive to keratinous substrate area and a rough area; an adhesive to keratinous substrate area and a colored area; an adhesive to keratinous substrate area and a shiny area; an adhesive to keratinous substrate area and a matte area; an adhesive to keratinous substrate area and a hydrophobic area; a reactive to keratinous substrate area and a UV filter area; a reactive to keratinous substrate area and a smooth area; a reactive to keratinous substrate area and a rough area; a reactive to keratinous substrate area and a colored area; a reactive to keratinous substrate area and a shiny area; a reactive to keratinous substrate area and a matte area; a reactive to keratinous substrate area and a hydrophobic area; or two reactive areas that react with each other.

Generally, as used herein, "adhesive" means tacky or sticky. "Reactive" as used herein, generally means reactive with other components by forming covalent bonds. For example, a reactive dye can be reactive to the hair or to another dye.

In another embodiment, the MPPs in accordance with the invention can be multiphasic, having at least two distinct, homogenous phases. The MPPs can have 2, 3, 4, 5, or 6 homogenous phases. In one embodiment, the MPPs of the invention include 2 phases, in another embodiment, the MPPs of the invention include 3 phases, and in still another embodiment the MPPs of the invention include 4 phases.

A phase of an MPP could also be produced such that it releases certain contained materials over time. Some regions may be reactive so that upon application of, for example, a developer, a color is generated, or a physical or chemical change occurs within that region causing a change in its properties. One region may be swellable when exposed to one particular solvent and another region swellable, but only when exposed to a different solvent.

MPPs in accordance with the present invention can also be made from materials that are known to exhibit certain cosmetic, personal care, and dermatological properties, such as smell, lubricity, deodorizing, film forming, emmolliency, moisturizing, and the like. They may also act as an emulsifier or compatibilize helping to bring together, in a useful way, disparate materials because of the close proximity of their divergent areas.

In another embodiment, the MPPs have an average particle size of about 1 nanometer to about 10 millimeters. In a preferred embodiment, the MPPs have an average particle size of about 10 nanometers to about 1 millimeter. In a more preferred embodiment, the MPPs have an average particle size of about 100 nanometers to about 200 microns using the techniques described herein. Particle size can be measured using a laser light scattering technique, such as the Malvern Mastersizer 2000. For dry powders of particles that are larger than 50 microns, especially those which cannot be easily suspended, particle size can be determined by sieving. The size of the particles are determined based on the longest dimension of the MPP.

By using an MPP in accordance with the present invention, one may be more easily able to tailor the degree of adhesiveness exhibited by these particles and the resulting formulations. Without wishing to be bound by any particular theory of operation, it is believed that the use of same can, in certain instances, even more effectively tailor the surface area that is adhesive. Moreover, by using MPPs in accordance with the present invention, it may be possible to reduce the overall amount of particular matter added to obtain this desired result.

It should also be noted, that MPPs in accordance with the present invention can be constructed with a generally nonadhesive material with discrete dots or regions imprinted or imparted thereon, which are of adhesive nature. See Figure 1, Particle K. By analogy, the black pentagonal areas of a soccer ball could be likened to adhesive patches distributed on the surface of an otherwise nonadhesive particle. By manipulating the number of adhesive patches and their placement, one may also be able to effect the geometry and interaction between various hair strands, for example, and these particles. This type of structure is not limited to adhesive phase containing MPPs.

Another desirable MPP can be a biphasic or multiphasic particle consisting of at least one hydrophilic phase or at least one hydrophobic phase. In addition, the particle could include an area or phase which is highly hydrophobic and another which is highly hydrophilic. The use of these particles could affect the overall hydrophobic lipophilic balance of a material or could act as a surfactant or compatibilizer between phases or materials contained within a personal care, cosmetic, topical or dermatological product, which would otherwise be nonmiscible.

Another example of particles in accordance with the present invention is a particle where one part is particularly reflective, containing for example a phase of a material such as silver, while the other area is colored. When placed in a hair care product these MPPs could provide both enhanced shine and color, tint, or highlight to the hair. Particles made of, or containing, two disparate pigmented or colored surface may also be useful where it is important to ensure that the two colors remain in close proximity such that the overall resulting color is tightly controlled. A mixture of such MPPs and, indeed, a mixture of different MPPs, each containing different colors either alone or in combination with other traditional pigments and colorants could provide uniquely rich color, unique color, overtones, or undertones or unique color and qualities.

In another embodiment, one phase could be tacky or an adhesive and another phase could contain a UV-blocking material. When this material is applied to the skin, the adhesive portion helps provide additional substantivity and ensures that the portion of the MPP, which is capable of providing UV-protection is pointing outwardly forming a barrier between the sun and the skin.

In other embodiments, MPPs can be made wherein one phase is inorganic and/or metallic or contains materials which are inorganic and/or metallic. A phase could be made of a silver or other metals, such as Zn, Ti, or any other metal, or mineral could be used. Metals include those selected from Groups IIA, IIB, VA, and IIIB through VIIIB of the periodic table of elements, regardless of oxidation state, and mixtures thereof. In a preferred embodiment of the present invention, the metals, and indeed metalcations may be selected from Be, Mg, Ca, Sr, Zn, Cd, Hg, N, P, Sb, Mn, Fe, Co, Ni, Cu, Mo, Pd, Ag, Au, Zr, and Ti regardless of oxidation state. In a most preferred embodiment of the present invention, the metal may be selected from Zn, Cu, Co, Fe, Au, and Ag.

As metal salts, metal oxides, or metal elements described above can also be used as constituitive of phase compounds. The anion of the metal salt may be selected from, without limitation, Cl₂⁻, SO₄²⁻, SO₃²⁻, PO₄³⁻, HPO₄²⁻, CrO₄²⁻, CR₂O₇²⁻, C₂O₄²⁻, S₂O₃²⁻, NO₃⁻, NO₂⁻, CIO₄⁻, CIO₃⁻, CIO₂⁻, OCl⁻, CH₃COO⁻, and IO₃⁻.

Generally however, MPPs of the present invention include at least one phase which is polymeric in nature and these polymers may be natural or synthetic. Examples of natural polymers which are contemplated include saccharides, oligosaccharides, polysaccharides, celluloses, modified celluloses, starches, modified starches, and chitosans. Synthetic polymers include natural and synthetic rubbers, urethanes, polyesters, polystyrenes, polycarbonates, polyacrylates, copolymers, block copolymers, thermoplastic resins, thermosets resins, polyolefins, and the like. Without limitation, these materials can include (meth)acrylate-based resins, styrene resins, polyamide-based resins, polyolefin-based resins, cellulose-based resins, silicon-based resins, and fluorine-based resins.

Any polymer that is available in, or can be placed in, a solution or suspension, preferably with water or a short chain alcohol (C₁-C₆), that has an appropriate molecular weight and an appropriate glass transition temperature (Tg) is contemplated to make the MPPs in accordance with the present invention. Generally, an appropriate molecular weight is from about 20,000 daltons to about 10,000,000 daltons. Generally, an appropriate Tg is from about -100°C to about 250°C.

Examples of the monomers that form styrene-based resins are alkyl styrenes such as methylstyrene, dimethylstyrene, trimethylstyrene, ethylstyrene, diethylstyrene, triethylstyrene, propylstyrene, butylstyrene, hexylstyrene, heptylstyrene, octylstyrene, or the like; a halogenated styrene such as fluorostyrene, chlorostyrene, bromostyrene, dibromostyrene, iodostyrene, chloromethylstyrene, or the like; nitrostyrene; acetylstyrene; methoxystyrene; and the like.

Examples of monomers that form (meth)acrylate-based resins are methyl (meth)acrylate, ethyl (meth)acrylate, propyl (meth)acrylate, butyl (meth)acrylate, 2-ethylhexyl (meth)acrylate, lauryl (meth)acrylate, stearyl (meth) acrylate, cyclohexyl (meth)acrylate, 2-hydroxyethyl (meth)acrylate, 2-propyl (meth)acrylate, chloro-2-hydroxyethyl (meth)acrylate, diethylene-glycol mono(meth)acrylate, methoxy ethyl (meth)acrylate, glycidyl (meth)acrylate, dicyclopentanyl (meth)acrylate, isobronol (meth)acrylate, and the like.

Fixing polymers may be used such as anionic, cationic, amphoteric, and nonionic fixing polymers and combinations thereof. As used herein in connection with the term polymer refers to homopolymers and copolymers, the copolymers being derived from more than one type of monomer.

The cationic fixing polymers comprise cationic moieties or moieties that are convertible to cationic moieties. Suitable examples of cationic fixing polymers, which can be used according to the present invention, are those that may be chosen from polymers comprising at least one group chosen from primary amine groups, secondary amine groups, tertiary amine groups, and quaternary amine groups, wherein the at least one group forms part of the polymer chain or is linked directly to it, having a weight average molecular weight ranging from about 500 to about 5,000,000, such as from about 100 to about 3,000,000.

Among these polymers, mention may be made more particularly of the following cationic fixing polymers:
(1) homopolymers and copolymers derived from monomers chosen from (meth)acrylic esters and (meth)acrylic amides comprising units of at least one of the following formulae: 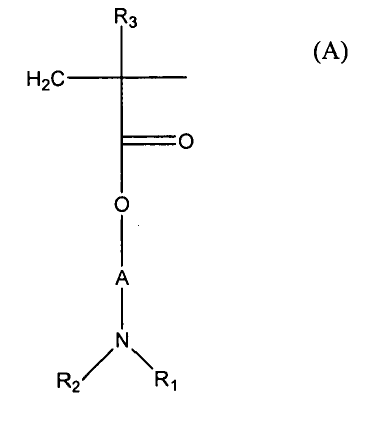 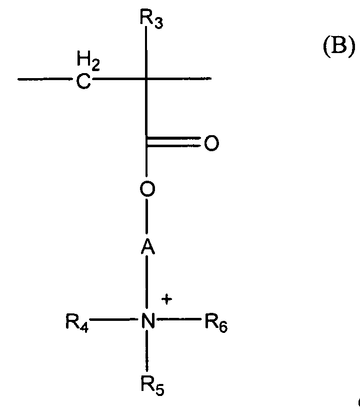 or 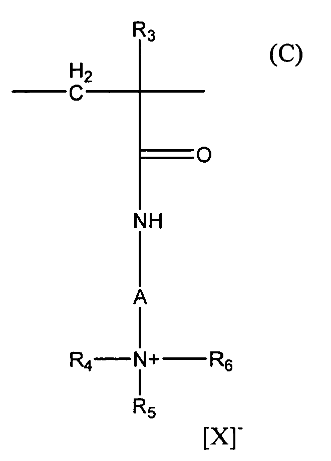
in which each R₃ is independently chosen from hydrogen and CH₃ groups; each A is independently chosen from linear and branched alkyl groups comprising 1 to 6 carbon atoms and hydroxyalkyl groups comprising 1 to 4 carbon atoms; each R₄, R₅, and R₆ is independently chosen from alkyl groups comprising 1 to 18 carbon atoms and benzyl groups; each R₁ and R₂ is independently chosen from hydrogen and alkyl groups comprising 1 to 6 carbon atoms; and each X⁻ is independently chosen from methyl sulfate anions and halide anions, such as chloride or bromide anions.

In one embodiment, the copolymers of family (1) further comprise at least one unit derived from monomers chosen from (meth)acrylamides, diacetone (meth)acrylamides, (meth)acrylamides substituted on the nitrogen by a group chosen from lower alkyls, (meth)acrylic acids, esters of (meth)acrylic acids, vinyllactams such as vinylpyrrolidone and vinyl-caprolactam, and vinyl esters.

Thus, mention may be made, among these cationic copolymers of the family
(1), of: copolymers of acrylamide and of dimethylaminoethyl methacrylate quaternized with dimethyl sulfate or with a dimethyl halide, such as that sold under the name Hercofloc by the company Hercules; copolymers of acrylamide and of methacryloyloxyethyltrimethylammonium chloride which are disclosed, for example, in EP-A-080,976, the disclosure of which relating to cationic polymers is incorporated herein by reference, and sold, for example, under the name Bina Quat P 100 by the company Ciba-Geigy; copolymers of acrylamide and of methacryloyloxyethyltrimethylammonium methosulfate, such as that sold under the name Reten by the company Hercules; optionally quaternized vinylpyrrolidone/dialkyl-aminoalkyl (meth)acrylate copolymers, which are disclosed, for example, in French Patents 2,077,143 and 2,393,573, the disclosures of which relating to cationic polymers are incorporated herein by reference, and sold, for example, under the name "Gafquat" by the company ISP, such as, for example, "Gafquat 734" or "Gafquat 755", or else the products named "Copolymer 845, 958 and 937"; dimethylaminoethyl methacrylate/vinylcaprolactam/vinylpyrrolidone terpolymers, such as the product sold under the name Gaffix VC 713 by the company ISP, and the quaternized vinylpyrrolidone/dimethylaminopropylmethacrylamide copolymer, such as the product sold under the name "Gafquat HS 100" by the company ISP;
(2) the quaternized polysaccharides, disclosed more particularly in U.S. Patent Nos. 3,589,578 and 4,031,307, the disclosures of which relating to quaternized polysaccharides polymers are incorporated herein by reference, such as guar gums comprising cationic trialkylammonium cationic groups. Such products are sold in particular under the trade names Jaguar C 13 S, Jaguar C 15, and Jaguar C 17 by the company Meyhall.
(3) quaternized copolymers of vinylpyrrolidone and of vinylimidazole, such as the products sold by BASF under the name Luviquat TFC.
(4) chitosans or their salts. The salts, which can be used, are in particular chitosan acetate, lactate, glutamate, gluconate, or pyrrolidone-carboxylate. Mention may be made, among these compounds, of the chitosan the name Kytan Crude Standard by the company Aber Technologies and the chitosan pyrrolidone-carboxylate sold under the name Kytamer PC by the company Amerchol.
(5) Cationic cellulose derivatives, such as the copolymers of cellulose and the cellulose derivatives grafted with a water-soluble quaternary ammonium monomer and disclosed in particular in U.S. Patent No. 4,131,576, the disclosure of which relating to cationic cellulose derivatives is incorporated herein by reference. Examples include hydroxyalkyl celluloses, for example hydroxymethyl, hydroxyethyl, and hydroxypropyl celluloses grafted in particular with a methacryloylethyltrimethylammonium, methacrylamidopropyltrimethylammonium, or diallyldimethylammonium salt.

The commercial products corresponding to this definition are more particularly the products sold under the name "Celquat L 200" and "Celquat H 100" by the company National Starch.

The anionic fixing polymers, which can be used according to the present invention, are polymers comprising groups derived from carboxylic, sulfonic, and/or phosphoric acid and having a weight average molecular weight ranging from about 500 to about 5,000,000.

The carboxyl groups may be contributed by unsaturated mono- or dicarboxylic acid monomers such as those corresponding to the formula: 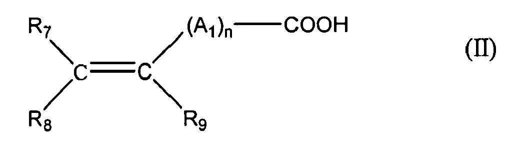 in which n is an integer ranging from 0 to 10; A₁ denotes a methylene group and when n is greater than 1, each A₁ is independently represented by --LCH₂ --, where L is chosen from a valency bond and heteroatoms, such as oxygen and sulfur; R₇ is chosen from hydrogen, phenyl groups, and benzyl groups; R₈ is chosen from hydrogen, lower alkyl groups, and carboxyl groups; and R₉ is chosen from hydrogen, lower alkyl groups, --CH₂--COOH groups, phenyl groups, and benzyl groups.

As defined herein, a lower alkyl group denotes a group having 1 to 4 carbon atoms, such as methyl and ethyl.

The anionic fixing polymers comprising carboxyl groups according to the invention may be chosen from:
A) Homopolymers and copolymers of (meth)acrylic acids or (meth)acrylic salts and in particular the products sold under the names Versicol E or K by the company Allied Colloid and Ultrahold by the company BASF, the copolymers of acrylic acid and of acrylamide sold in the form of their sodium salt under the names Reten 421, 423, or 425 by the company Hercules, and the sodium salts of polyhydroxycarboxylic acids.
B) Copolymers of (meth)acrylic acid with a monoethylenic monomer, such as ethylene, styrene, vinyl esters, and (meth)acrylic acid esters, optionally grafted onto a polyalkylene glycol, such as polyethylene glycol, and optionally crosslinked. Such polymers are disclosed in particular in French Patent 1,222,944 and German Application 2,330,956, the disclosures of which relating to such copolymers are incorporated herein by reference. The copolymers of this type comprising, in their chain, an optionally N-alkylated and/or hydroxyalkylated acrylamide unit, such as disclosed in particular in Luxembourg Patent Applications 75370 and 75371, the disclosures of which relating to such copolymers are incorporated herein by reference, or sold under the name Quadramer by the company American Cyanamid. Mention may also be made of copolymers of acrylic acid and of C₁ -C₄ alkyl methacrylate and terpolymers of vinylpyrrolidone, of acrylic acid, and of C₁ -C₂₀ alkyl methacrylate for example lauryl methacrylate, such as that sold by the company ISP under the name Acrylidone LM, and methacrylic acid/ethyl acrylate/tert-butyl acrylate terpolymers, such as the product sold under the name Luvimer 100 P by the company BASF.
C) copolymers derived from crotonic acid, such as those comprising, in their chain, vinyl acetate or propionate units and optionally other monomers, such as (meth)allyl esters, vinyl ether or vinyl ester of a linear or branched saturated carboxylic acid comprising a long hydrocarbon chain, such as those comprising at least 5 carbon atoms, it optionally being possible for these polymers to be grafted and crosslinked, or alternatively a vinyl, allyl, or methallyl ester of an α- or β-cyclic carboxylic acid. Such polymers are disclosed, inter alia, in French Patents 1,222,944, 1,580,545, 2,265,782, 2,265,781, 1,564,110, and 2,439,798, the disclosures of which relating to copolymers of crotonic acid are incorporated herein by reference. Commercial products coming within this class are the Resins 28-29-30, 26-13-14, and 28-13-10 sold by the company National Starch.
D) copolymers derived from C₄ -C₈ monounsaturated carboxylic acids or anhydrides chosen from: copolymers comprising units derived from
   (i) at least one monomer chosen from maleic, fumeric, and itaconic acids and anhydrides thereof and
   (ii) at least one monomer chosen from vinyl esters, vinyl ethers, vinyl halides, phenylvinyl derivatives, acrylic acids, and acrylic acid esters, the anhydride functional groups of these copolymers optionally being monoesterified or monoamidated. Such polymers are disclosed in particular in U.S. Patent Nos. 2,047,398, 2,723,248, and 2,102,112 and GB 839,805, the disclosures of which relating to such copolymers are incorporated herein by reference, and in particular those sold under the names Gantrez AN or ES by the company ISP. copolymers comprising units derived from
      (i) at least one monomer chosen from maleic, citraconic, and itaconic anhydrides and
      (ii) at least one monomer chosen from (meth)allyl esters, optionally comprising in their chain at least one unit derived from groups chosen from (meth)acrylamide, α-olefin, (meth)acrylic ester, (meth)acrylic acid, and vinylpyrrolidone groups. The anhydride functional groups of these copolymers optionally are monoesterified or monoamidated. These polymers are, for example, disclosed in French Patents 2,350,384 and 2,357,241 the disclosures of which relating to such copolymers are incorporated herein by reference.
E) polyacrylamides comprising carboxylate groups. (2) The anionic fixing polymers comprising sulfonic groups may be chosen from polymers comprising units, such as those derived from vinylsulfonic, styrenesulfonic, naphthalenesulfonic, and acrylamidoalkylsulfonic acids and their derivatives. These polymers may be chosen from: salts of polyvinylsulfonic acid having a weight average molecular weight that ranges from about 1000 to about 100,000, as well as the copolymers derived from at least one unsaturated comonomer, such as acrylic and methacrylic acids, their esters, acrylamides, their derivatives, vinyl ethers, and vinylpyrrolidone; salts of polystyrenesulfonic acid, the sodium salts having a weight average molecular weight ranging from about 100,000 to about 500,000, which are sold respectively under the names Flexan 500 and Flexan 130 by National Starch. These compounds are disclosed in Patent FR 2,198,719, the disclosure of which relating to salts of polystyrenesulfonic acid is incorporated herein by reference; salts of polyacrylamidesulfonic acids, including those mentioned in U.S. Patent No. 4,128,631, the disclosure of which relating to salts of polyacrylamidesulfonic acid is incorporated herein by reference, and more particularly the polyacrylamidoethylpropanesulfonic acid sold under the name Cosmedia Polymer HSP 1180 by Henkel.

In one embodiment, the anionic fixing polymers are chosen from acrylic acid copolymers, such as the acrylic acid/ethyl acrylate/N-tert-butylacrylamide terpolymer sold under the name Ultrahold Strong by the company BASF; copolymers derived from crotonic acid, such as the vinyl acetate/vinyl tert-butyl-benzoate/crotonic acid terpolymers and the crotonic acid/vinyl acetate/vinyl neododecanoate terpolymers sold under the name Resin 28-29-30 by the company National Starch; polymers derived from at least one monomer chosen from maleic, fumeric, and itaconic acids and anhydrides thereof and also from at least one monomer chosen from vinyl esters, vinyl ethers, vinyl halides, phenylvinyl derivatives, acrylic acid, and esters of acrylic acid, such as the monoesterified methyl vinyl ether/maleic anhydride copolymer sold under the name Gantrez ES 425 by the company ISP; copolymers of methacrylic acid and of methyl methacrylate sold under the name Eudragit L by the company Rohm Pharma; the copolymer of methacrylic acid and of ethyl acrylate sold under the name Luvimer MAEX or MAE by the company BASF; the vinyl acetate/crotonic acid copolymer sold under the name Luviset CA 66 by the company BASF; and the vinyl acetate/crotonic acid copolymer grafted by polyethylene glycol sold under the name Aristoflex A by the company BASF.

In another embodiment, the anionic fixing polymers are chosen from the monoesterified methyl vinyl ether/maleic anhydride copolymer sold under the name Gantrez ES 425 by the company ISP; the acrylic acid/ethyl acrylate/N-tert-butylacrylamide terpolymer sold under the name Ultrahold Strong by the company BASF; the copolymers of methacrylic acid and of methyl methacrylate sold under the name Eudragit L by the company Rohm Pharma; the vinyl acetate/vinyl tert-butylbenzoate/crotonic acid terpolymers and the crotonic acid/vinyl acetate/vinyl neododecanoate terpolymers sold under the name Resin 28-29-30 by the company National Starch; the copolymer of methacrylic acid and of ethyl acrylate sold under the name Luvimer MAEX or MAE by the company BASF; and the vinyl-pyrrolidone/acrylic acid/lauryl methacrylate terpolymer sold under the name Acrylidone LM by the company ISP.

The amphoteric fixing polymers, which can be used in accordance with the invention, may be chosen from polymers comprising X and Y units, distributed randomly in the polymer chain, where the X unit is chosen from units derived from at least one monomer comprising at least one basic function, in particular a basic nitrogen atom, and where the Y unit is chosen from units derived from at least one acidic monomer comprising at least one group chosen from carboxyl groups and sulfo groups, or else where each X and Y unit is independently chosen from groups derived from zwitterionic carboxybetaine and sulfobetaine monomers. In another embodiment, the amphoteric fixing polymers, which can be used in accordance with the invention, may be chosen from polymers comprising X and Y units, each X and Y unit is independently chosen from at least one cationic polymer chain comprising at least one group chosen from primary amine groups, secondary amine groups, tertiary amine groups, and quaternary amine groups, in which at least one of the amine groups comprises a group chosen from carboxyl and sulfo groups linked by way of a hydrocarbon group, or else the X and Y units, which may be different or identical, form part of a chain of at least one polymer comprising an α,β-dicarboxy ethylene unit, wherein at least one of the carboxyl groups has been reacted with a polyamine comprising at least one group chosen from primary and secondary amine groups.

In one embodiment, the amphoteric fixing polymers corresponding to the definition given above are chosen from the following polymers:
(1) polymers resulting from the copolymerization of a monomer derived from a vinyl compound carrying a carboxyl group, such as (meth)acrylic acids, maleic acids, and α-chloracrylic acids, and of a basic monomer derived from a substituted vinyl compound comprising at least one basic atom, such as dialkylaminoalkyl (meth)acrylate and dialkylaminoalkyl (meth)acrylamide. Such compounds are disclosed in U.S. Patent No. 3,836,537, the disclosure of which relating to amphoteric polymers is incorporated herein by reference.
(2) polymers comprising units derived from:
   a) at least one monomer chosen from (meth)acrylamides substituted on the nitrogen with an alkyl group,
   b) at least one acidic comonomer comprising at least one reactive carboxylic group, and
   c) at least one basic comonomer, such as esters comprising at least one substituent chosen from primary, secondary, tertiary, and quaternary amine substituents of (meth)acrylic acids, and the product of quaternization of dimethylaminoethyl methacrylate with dimethyl or diethyl sulfate.

The at least one N-substituted (meth)acrylamide monomer recited in (a) is more particularly chosen from N-substituted (meth)acrylamides, wherein the alkyl groups comprise from 2 to 12 carbon atoms, such as N-ethylacrylamide, N-tert-butylacrylamide, N-tert-octylacrylamide, N-octylacrylamide, N-decylacrylamide, N-dodecylacrylamide, and the corresponding methacrylamides.

The at least one acidic comonomer recited in (b) is more particularly chosen from (meth)acrylic acids, crotonic acids, itaconic acids, maleic acids, fumeric acids, C₁ -C₄ alkyl monoesters of maleic acid, C₁ -C₄ alkyl monoesters of fumeric acid, C₁ -C₄ alkyl monoesters of maleic anhydride, and C₁ -C₄ alkyl monoesters of fumeric anhydride.

The at least one basic comonomer recited in (c) is more particularly chosen from aminoethyl, butylaminoethyl, N,N'-dimethylaminoethyl, and N-tert-butylaminoethyl methacrylates.

In one embodiment, the amphoteric fixing polymer is chosen from the copolymers for which the CTFA name (4th Ed., 1991) is octylacrylamide/acrylates/butylaminoethyl methacrylate copolymer, such as the products sold under the name Amphomer or Lovocryl 47 by the company National Starch.
(3) cross-linked and alkylated polyamino amides partially or totally derived from polyamino amides of general formula:

   ⁅ CO-R₁₀-CO-Z⁆ (III)

   in which R₁₀ represents a divalent group derived either from a saturated dicarboxylic acid, from an aliphatic mono- or dicarboxylic acid comprising an ethylenic double bond, from an ester of a lower alkanol having 1 to 6 carbon atoms of these acids, or from a group derived from the addition of any one of the said acids with a bisprimary or bissecondary amine; and Z denotes a group of a bisprimary, mono- or bissecondary polyalkylenepolyamine and, for example, represents:
   a) in the proportions of from about 60 mol% to 100 mol%, the group:

      -NH⁅(CH₂)ₓ-NH⁆ₚ (IV)

      where x=2 and p=2 or 3, or else x=3 and p=2 and where this group derives from diethylenetriamine, triethylenetetraamine, or dipropylenetriamine;
   b) in the proportions of from 0 mol % to about 40 mol %, the above group (IV), in which x=2 and p=1 and which derives from ethylenediamine, or the group derived from piperazine: 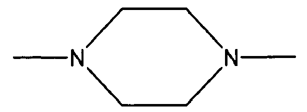
   c) in the proportions of from 0 mol % to about 20 mol %, the -NH- (CH₂)₆ -NH- group derived from hexamethylenediamine, these polyamino amines being crosslinked by addition of a difunctional crosslinking agent chosen from epihalohydrines, diepoxides, dianhydrides and bis-unsaturated derivatives, using from about 0.025 mol to about 0.35 mol of crosslinking agent per amine group of the polyamino amide and alkylated by the action of acrylic acid, chloroacetic acid or an alkane sultone, or salts thereof.

In one embodiment, the saturated carboxylic acids are chosen from acids having 6 to 10 carbon atoms, such as adipic acid, 2,2,4-trimethyladipic acid, 2,4,4-trimethyladipic acid, terephthalic acid, and acids comprising an ethylenic double bond such as, for example, acrylic acid, methacrylic acid and itaconic acid.

In one embodiment, the alkane sultones used in the alkylation are chosen from propane sultone and butane sultone and the salts of the alkylating agents are chosen from sodium and potassium salts.
(4) polymers comprising zwitterionic units of formula: 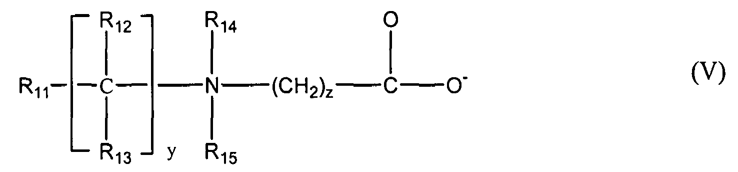 in which R₁₁ is chosen from polymerizable unsaturated groups such as a (meth)acrylate and (meth)acrylamide groups; y and z are independently chosen from integers ranging from 1 to 3; R₁₂ and R₁₃ are independently chosen from hydrogen, methyl groups, ethyl groups, and propyl groups; R₁₄ and R₁₅ are independently chosen from hydrogen and alkyl groups, wherein the sum of the carbon atoms in R₁₄ and R₁₅ is less than or equal to 10.

The polymers comprising such units may further comprise units derived from non-zwitterionic monomers, such as dimethylaminoethyl (meth)acrylate, diethylaminoethyl (meth)acrylate, alkyl (meth)acrylates, (meth)acrylamides, and vinyl acetates.

Mention may be made, by way of example, of the methyl methacrylate/ methyl dimethylcarboxymethylammonioethyl methacrylate copolymer, such as the product sold under the name Diaformer Z301 by the company Sandoz.
(5) polymers derived from chitosan comprising monomer units corresponding to the following formulae: 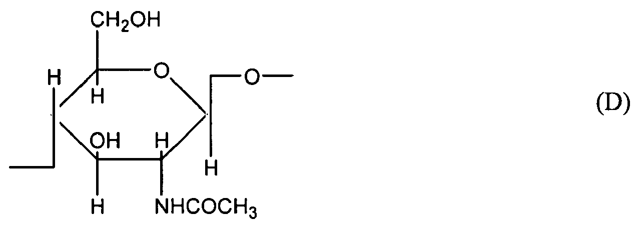 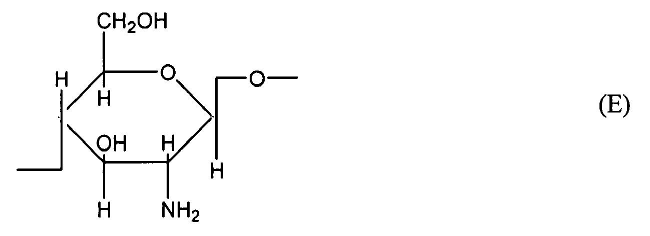 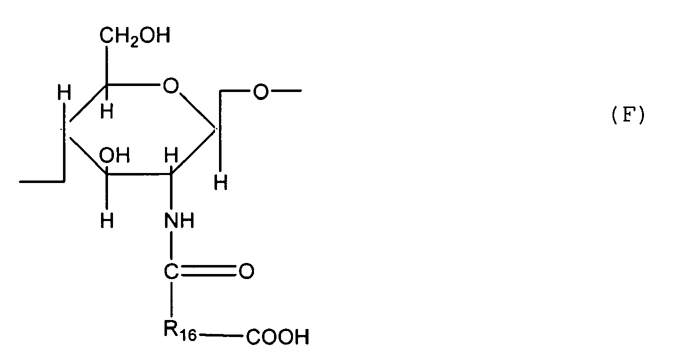 the unit D being present in proportions ranging from 0% to about 30%, the unit E in proportions ranging from about 5% to about 50% and the unit F in proportions ranging from about 30% to about 90%, it being understood that, in this unit F, R₁₆ represents a group of formula: 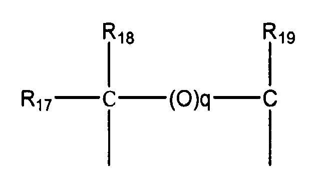 in which, if q=0, R₁₇, R₁₈, and R₁₉, which are identical or different, are chosen from hydrogen, methyl groups, hydroxyl groups, acetoxy groups, amino residues, monoalkylamine residues, and dialkylamine residues, optionally interrupted by one or more nitrogen atoms and/or optionally substituted by one or more amine, hydroxyl, carboxy, alkylthio, or sulfo groups, and alkylthio residues in which the alkyl group carries an amino residue, at least one of R₁₇, R₁₈, and R₁₉ being, in this case, hydrogen; or, if q=1, R₁₇, R₁₈, and R₁₉ each represent hydrogen, and the salts formed by these compounds with bases or acids.
(6) Polymers derived from the N-carboxyalkylation of chitosan, such as the N-(carboxymethyl)chitosan or the N-(carboxybutyl)chitosan sold under the name "Evalsan" by the company Jan Dekker.
(7) Polymers corresponding to the general formula (VI), for example disclosed in French Patent 1,400,366, the disclosure of which relating to amphoteric polymers is incorporated herein by reference: 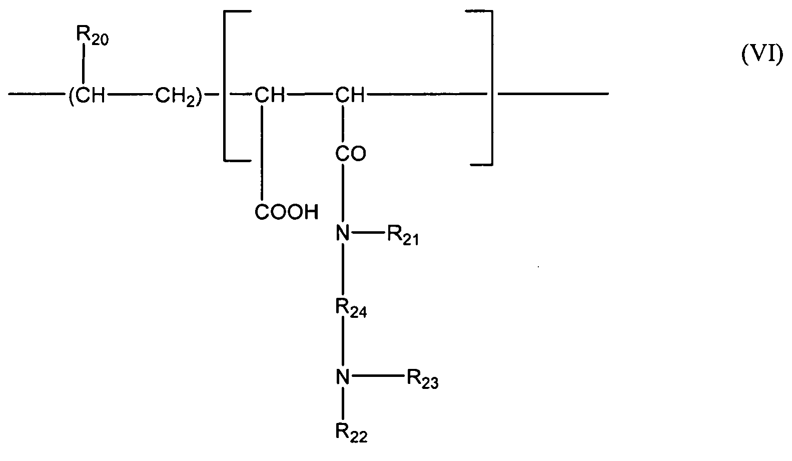 in which R₂₀ is chosen from hydrogen, CH₃ O, CH₃ CH₂ O, and phenyl groups; R₂₁ is chosen from hydrogen and lower alkyl groups such as methyl or ethyl; R₂₂ is chosen from hydrogen and lower alkyl groups such as methyl or ethyl; and R₂₃ is chosen from lower alkyl groups such as methyl or ethyl and groups corresponding to the formula: -R₂₄ -N(R₂₂)₂, where R₂₄ is chosen from -CH₂ -CH₂ -, -CH₂ -CH₂ -CH₂ -, and -CH₂ -CH(CH₃) - groups and R₂₂ is the same as above, and the higher homologues of these groups comprising up to 6 carbon atoms.
(8) Amphoteric fixing polymers of the -D-X-D-X- type chosen from:
   a) polymers obtained by reaction of chloracetic acid or sodium chloracetate with compounds comprising at least one unit of formula:

      -D-X-D-X-D- (VII)

      where D denotes a group 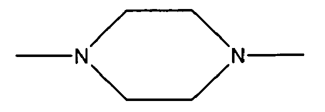 and X denotes the symbol E or E', E and E', which are identical or different, denote a divalent group chosen from straight- and branched-chain alkylene groups comprising up to 7 carbon atoms in the main chain, which is unsubstituted or substituted by hydroxyl groups and which can additionally comprise oxygen, nitrogen, or sulfur atoms or 1 to 3 aromatic and/or heterocyclic rings; the oxygen, nitrogen, and sulfur atoms being present in the form of ether, thioether, sulfoxide, sulfone, sulfonium, alkylamine or alkenylamine groups, benzylamine, amine oxide, quaternary ammonium, amide, imide, alcohol, ester, and/or urethane groups.
   b) Polymers of formula:

      -D-X-D-X- (VII')

      in which D denotes a group 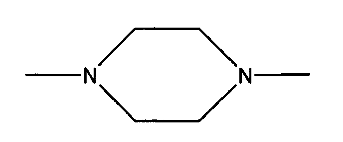 and X denotes the symbol E or E' and E' at least once, where E has the meaning indicated above and E' is a divalent group chosen from straight- and branched-chain alkylene groups having up to 7 carbon atoms in the main chain, which is substituted or unsubstituted by one or more hydroxyl groups and which comprises one or more nitrogen atoms, the nitrogen atom being substituted by an alkyl chain optionally interrupted by an oxygen atom and necessarily comprising one or more carboxyl functional groups or one or more hydroxyl functional groups and wherein the polymer of formula VII' is betainized by reaction with chloracetic acid or sodium chloracetate.
(9) (C₁ -C₅)alkyl vinyl ether/maleic anhydride copolymers, which are partially modified by semiamidation with an N,N-dialkylaminoalkylamine, such as N,N-dimethylaminopropylamine, or by semiesterification with an N,N-dialkanolamine. These copolymers can also comprise other vinyl comonomers, such as vinylcaprolactam.

In one embodiment, the amphoteric fixing polymers according to the invention are chosen from family (3), such as the copolymers with the CTFA name (4^{th} Ed. 1991) of octylacrylamide/acrylates/butylaminoethyl methacrylate copolymer, such as the products sold under the names Amphomer, Amphomer LV 71, or Lovocryl 47 by the company National Starch, and family (4), such as the copolymer of methyl methacrylate/dimethyl carboxymethylammonio methyl ethylmethacrylate, sold, for example, under the name Diaformer Z301 by the company Sandoz.

The nonionic fixing polymers, which can be used according to the present invention, are chosen, for example, from: vinylpyrrolidone homopolymers; copolymers of vinylpyrrolidone and of vinyl acetate; polyalkyloxazolines, such as the polyethyloxazolines sold by the company Dow Chemical under the names PEOX 50 000, PEOX 200 000 and PEOX 500 000; vinyl acetate homopolymers, such as the product sold under the name Appretan EM by the company Hoechst or the product sold under the name Rhodopas A 012 by the company Rhone-Poulenc; copolymers of vinyl acetate and of acrylic ester, such as the product sold under the name Rhodopas AD 310 by Rhone-Poulenc; copolymers of vinyl acetate and of ethylene, such as the product sold under the name Appretan TV by the company Hoechst; copolymers of vinyl acetate and of maleic ester, for example of dibutyl maleate, such as the product sold under the name Appretan MB Extra by the company Hoechst; copolymers of polyethylene and of maleic anhydride; alkyl acrylate homopolymers and alkyl methacrylate homopolymers, such as the product sold under the name Micropearl RQ 750 by the company Matsumoto or the product sold under the name Luhydran A 848 S by the company BASF; acrylic ester copolymers such as, for example, copolymers of alkyl (meth)acrylates, such as the products sold by the company Rohm & Haas under the names Primal AC-261 K and Eudragit NE 30 D, by the company BASF under the names Acronal 601, Luhydran LR 8833 or 8845, and by the company Hoechst under the names Appretan N 9213 or N 9212; copolymers of acrylonitrile and of a nonionic monomer chosen, for example, from butadiene and alkyl (meth)acrylates; mention may be made of the products sold under the names Nipol LX 531 B by the company Nippon Zeon or those sold under the name CJ 0601 B by the company Rohm & Haas; polyurethanes, such as the products sold under the names Acrysol RM 1020 or Acrysol RM 2020 by the company Rohm & Haas, and the products Uraflex XP 401 UZ and Uraflex XP 402 UZ by the company DSM Resins; copolymers of alkyl acrylate and of urethane, such as the product 8538-33 by the company National Starch; polyamides, such as the product Estapor LO 11 sold by the company Rhone-Poulenc.; nonionic guar gums that are chemically modified or unmodified.

The unmodified nonionic guar gums are, for example, the products sold under the name Vidogum GH 175 by the company Unipectine and under the name Jaguar C by the company Meyhall.

The modified nonionic guar gums, which may be used according to the invention, are, for example, modified with C₁-C₆ hydroxyalkyl groups. Examples, which may be mentioned, are hydroxymethyl, hydroxyethyl, hydroxypropyl, and hydroxybutyl groups.

These guar gums are well known in the prior art and may be prepared, for example, by reacting corresponding alkene oxides such as, for example, propylene oxides with guar gum so as to obtain a guar gum modified with hydroxypropyl groups.

Such nonionic guar gums, optionally modified with hydroxyalkyl groups, are sold, for example, under the trade names Jaguar HP8, Jaguar HP60, Jaguar HP120, Jaguar DC 293 and Jaguar HP 105 by the company Meyhall and under the name Galactosol 4H4FD2 by the company Aqualon.

The alkyl groups in the nonionic polymers comprise from 1 to 6 carbon atoms, except where otherwise mentioned.

According to the invention, it is also possible to use fixing polymers of grafted silicone type comprising a polysiloxane portion and a portion comprising a non-silicone organic chain, one of the two portions constituting the main chain of the polymer and the other being grafted onto the said main chain. These polymers are disclosed, for example, in EP-A-0,412,704, EP-A-0,412,707, EP-A-0,640,105, WO 95/00578, EP-A-0,582,152, and WO 93/23009 and U.S. Patent Nos. 4,693,935, 4,728,571, and 4,972,037, the disclosures of which relating to grafted silicone type polymers are incorporated herein by reference. These polymers are, for example, anionic or nonionic.

Such polymers are, for example, copolymers which can be obtained by radical polymerization from the monomer mixture comprising:
a) about 50% to about 90% by weight of tert-butyl acrylate;
b) 0% to about 40% by weight of acrylic acid;
c) about 5% to about 40% by weight of silicone macromonomer of formula: 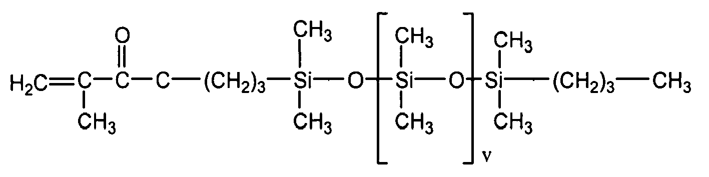
where v is a number ranging from 5 to 700; the percentages by weight being calculated with respect to the total weight of the monomers.

Other examples of grafted silicone polymers are, in particular, polydimethylsiloxanes (PDMSs) onto which are grafted, via a thiopropylene-type connecting chain, mixed polymer units of the poly (meth) acrylic acid type and of the poly(alkyl (meth)acrylate) type and polydimethylsiloxanes (PDMSs) onto which are grafted, via a thiopropylene-type connecting chain, polymer units of the poly(isobutyl (meth)acrylate) type.

It is also possible to use, as fixing polymers, functionalized or non-functionalized and silicone-comprising or non-silicone-comprising polyurethanes.

Examples of useful polyurethanes include those disclosed in Patents EP 0,751,162, EP 0,637,600, FR 2,743,297, EP 0,648,485, EP 0,656,021, WO 94/03510, and EP 0, 619, 111, the disclosure of which relating to polyurethanes are incorporated herein by reference.

In a further embodiment, the fixing polymers may be used in solubilized form or may be in the form of dispersions of solid particles (latex or pseudo-latex).

In another embodiment, the at least one adhesive of the at least partial coating and/or the at least one substrate of the invention may be chosen from polymeric adhesives, such as (meth)acrylic copolymers comprising: (a) units derived from at least one monomer present at from about 0.1 to about 99% by weight, such as about 9 to about 99% by weight of the total weight of the polymer and (b) units derived from at least one co-monomer present at up to about 99.9% by weight, such as up to about 91% by weight.

The at least one monomer recited in (a) can generally be represented by formula: 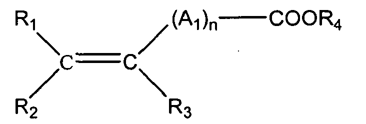 in which n is an integer ranging from 0 to 10; A.₁ denotes a methylene group and when n is greater than 1, each A₁ is independently represented by - LCH₂ -, where L is chosen from a valency bond and heteroatoms, such as oxygen and sulfur; R₁ is chosen from hydrogen, phenyl groups, and benzyl groups; R₂ is chosen from hydrogen, lower alkyl groups, and carboxyl groups; and R₃ is chosen from hydrogen, lower alkyl groups, - CH₂ - COOH groups, phenyl groups, and benzyl groups; and R₄ is chosen from hydrogen, C₁ to C₁₈ alkyls, C₂ to C₈ alkoxyalkyls, C₂ to C₈ alkylthioalkyls, and C₂ to C₈ cyanoalkyls.

The at least one monomer recited in (a) may, for example, be chosen from acrylic acids, methacrylic acids, salts thereof, and derivatives thereof, such as acrylic and methacrylic esters, including methyl acrylate, ethyl acrylate, n-butyl acrylate, isobutyl acrylate, hexyl acrylate, octyl acrylate, 2-ethylhexyl acrylate, decyl acrylate, methoxyacrylate, ethoxyacrylate, methylthiomethyl acrylate, and cyanopropyl acrylate.

The at least one co-monomer recited in (b) may contain one or more terminal CH₂ =C groups, for instance, acrylic or methacrylic esters such as methyl methacrylate, ethyl methacrylate, n-butyl methacrylate, and methyl methacrylate; vinyl halides such as vinyl chlorides; vinyl or allyl esters such as vinyl acetate, vinyl butyrate, and vinyl chloroacetate; and aromatic vinyls such as styrene, vinyltoluene, chloromethylstyrene, and vinylnaphthalene.

In a further embodiment, these (meth)acrylic copolymers may further comprise (c) units derived from at least one vinylidene co-monomer containing at least one group chosen from carboxyl and hydroxyl groups present at from about 1 to about 10% by weight.

Among the at least one vinylidene co-monomer recited in (c) containing at least one hydroxyl group, one may use, for example, acrylate monomers with a terminal hydroxyl group, such as hydroxyethyl acrylate, hydroxyethyl methacrylate, hydroxypropyl acrylate or some other hydroxymethylated diacetone acrylamide derivatives such as N-methylol acrylamide, N-methylol maleamide, N-propanolacrylamide, N-methylol methacrylamide or N-methylol-p-vinyl benzamide. The at least one vinylidene co-monomer recited in (c) containing at least one carboxyl group may be chosen from, for instance, acrylic acid, methacrylic acid, itaconic acid, citraconic acid, crotonic acid, and maleic acid.
MPPs can be formed in a number of known ways. See, for example, Millman et al., "Anisotropic particles synthesis in dielectrophoretically controlled microdroplet reactors", Nature Materials, Vol 4, Jan. 2005, 98-102, Reculusa et al., "Dissymmetrical Nanoparticles", Dekker Encyclopedia of Nanoscience and Nanotechnology, 2004, Marcel Dekker, Inc. 270 Madison Ave. NYC. MY. 10016, 943-53, Roh, et al., "Biphasic Janus particles with nanoscale anisotropy", Nature Materials, Vol 4, Oct. 2005, 759-763, and Perro, et al., "Design and synthesis of Janus micro- and nanoparticles", J. Mater. Chem., 2005, 15, 3745-3760, the complete texts of which are incorporated by reference for their teachings of how MPPs are made and a copy of each which are attached hereto as a portion of the application. See also Duguet (Centre de Recherche Paul Pascal, Université de Bordeaux - France), S. Reculusa, C. Poncet-Legrant, A. Perro, E. Duguet, E. Bourgeat-Lami, C. Mingotaud, S. Ravaine, Chem Mater, 17 (13) (2005) 3339, Growth of a nodule of PS on a silica particle; Casagrande (Laboratoire de Physique de la Matière Condensée, Collège de France, Paris - France), C. Casagrande, P.Fabre, E.Raphaël, M.Veyssié, Europhys Lett, 9(30) (1989) 253, Glass particle coated with two different materials on each side; Müller (Makromolekulare Chemie II, Physicalische Chemie II, Universität Bayreuth, Bayreuth - Germany), H.Xu, R.Erhardt, V.Abetz, A.Müller, W.Gedel, Langmuir, 17(22) (2001) 6787; R. Erhart, M. Zhang, A.Böker, H. Zettl, C. Abetz, P. Frederik, G. Krausch, V.Abetz, A. Müller, JACS, 125 (2003) 3260-67; R.Erhardt, A. Boeker, H.Ettl, H.Kaya, W.Pyckhout-Hintzen, G. Krausch, V.Abetz, A. Müller, Macromolecules, 34(4) (2001) 1069 Tribloc PS-PbA-PMMA, One bloc forms the core and the other two blocs phase separate; Saito, R.Saito, A.Fujita, A.Ichimura, K.Ishizu, J of Polym Sci A Polym Chem, 38(11), (2000) 2091-7 PS-PBMA + PVP as a cross linker, Phase separation of two blocs; Cohen, Z.Li, D.Lee. M.Rubner, R.Cohen, Macromolecules, 38 (2005) 7876, Core + double shell PAA and PAAm, the complete texts of which are incorporated by reference herein.

The various MPP structures, including without limitation, Janus particles, and the like disclosed in these references are also considered part of this invention. Also included herein are multiphasic particles useful for cosmetics, personal care products, and topical or dermatological products made by any of the processes described in the articles listed above.

In one process, biphasic MPPs are made by the simultaneous electrohydrodynamic jetting or spraying of parallel streams of polymers or polymer containing solutions under the influence of an electric field. In this embodiment, a laminar flow of two distinct polymer streams was pumped at suitable flow rates, typically in the range of a microliters per min ⁻¹ through a nozzle with a side-by-side geometry. This maintains a bipolar polymer/polymer interface between the two jetting materials throughout the nozzle until the electrified field influence particle creation. This biphasic character continued through the resulting droplet which becomes a MPP upon solidification. The morphology of the final particle is determined by, among other things, the viscosity, conductivity and surface tension of the materials used as well as the electrohydrodynamic parameters such as applied field strengths, flow rates and the like which were used.

It is also worthy of note that the MPP's of the present invention can include other materials as well, including, without limitation, materials that affect conductivity, viscosity, surface tension, and the like which can have an influence on the processability of the materials being used. Various solvents, solubilizers, compatabilizers, and stabilizers may also be used, although some of these, such as solvents, may be driven or dried and therefore not remain in the final MPP.

The factors which will influence the amount of MPPs used in a product in accordance with the present invention include, without limitation, the composition of the MPP and their functions, the product, the remaining ingredients within the product, the size of the MPPs, whether it is intended that they would be visible or not and the like.

Generally, the amount of MPPs in a given product will range from about between 0.01 to about 60 percent by weight of the finished product. In another embodiment they range from about between 0.10 to about 30 percent by weight, and in still another embodiment between about 0.50 and about 15 percent by weight.

In one embodiment, these products comprise MPPs in an amount of between about 0.01 and about 60 percent by weight of the composition and at least one carrier or additional ingredient. In another embodiment, these formulations also include both a carrier and an additional ingredient.

In another embodiment, the present invention relates to hair cleaning, conditioning, nourishing, treatment and coloring products, such as shampoos, conditioners, leave in conditioners, permanent and semi-permanent dyes, which comprise MPPs in an amount of between about 0.01 and about 60 percent by weight of the composition and at least one of a carrier and an additional ingredient.

Products in accordance with the present invention include all previously known compositions in the cosmetic, personal care, and topical or dermatological areas - whether or not such products currently or previously contain particulate. Without limitation, cosmetic products also referred to interchangeably herein as colored cosmetic products include materials used for coloring, shadowing, highlighting, or concealing areas of the skin. These include products which impart a tint, or change the optical properties of the substrate, such as providing a shine, sparkle, or matt finish. These include, without limitations, foundation, blush, rouge, eye pencils, eye liners, lip pencils, lip liners, lipsticks, nail polish, foundation, blush, bronzing products, eye shadow, lip gloss, and the like. Other cosmetic products may include fragrance. Personal care products include, without limitation, soaps, deodorants, moisturizers, shampoos, conditioners (leave in and wash off), styling products such as mousses, styling gels, styling sprays, depilatories, permanent waving solutions, permanent, semipermanent, or washout hair dyes, exfoliants, cream rinses, powders such as baby powder or talcum powder or corn starch, as well as UV-protecting agents such as sunscreens, sun tanning products, and the like. Topical preparations include antibacterial and antifungal agents, and inclusive protective materials. Dermatological preparations include, for example, moisturizers. The term "cosmetic composition" includes, without limitation, any and all cosmetics, personal care products, topical preparations, and dermatological preparations.

These products may be provided in any format including a solution (in which case the MPPs are generally suspended therein), a dispersion, a suspension, an emulsion, a colloid, a stick or pencil, a gel, a milk, a cream, a slurry, a powder, and the like. Thus MPPs in accordance with the present invention can be mixed, blended, or otherwise formulated with any number of materials currently used in the topical dermatology, personal care, or cosmetic industries.

The compositions of the present invention may further comprise at least one suitable (e.g., cosmetically or dermatologically acceptable) carrier and/or at least one additional ingredient. The additional ingredients of the compositions of the invention may also include one or more absorbents, anti-acne agents, anti-perspirants, antiwrinkles, anticaking agents, antifoaming agents, antimicrobial agents, antioxidants, antidandruff agents, binders, buffers, biological additives, bulking agents, chelating agents, conditioners, colorants, astringents, biocides, denaturants, detergents, dispersants, external analgesics, film formers, UVA filters, UVB filters, foaming agents, fragrance components, humectants, keratolytics, opacifying agents, pH adjusters, preservatives, propellants, proteins, retinoids, reducing agents, oxidizing agents, sequestrants, skin bleaching agents, skin-conditioning agents (humectants, miscellaneous, and occlusive), skin soothing agents, skin healing agents, hair bleaching agents, direct hair dyes, oxidation hair dyes, hair-conditioning agents (humectants, quats), hair repairing agents, hair fixative agents, softeners, solubilizing agents, lubricants, penetrants, plasticizers, solvents and co-solvents, sunscreening additives, salts, essential oils, thickeners, volatile or nonvolatile silicones, plasticizers dyestuffs, and vitamins.

Depending on the application, the amount of additional ingredient(s) present in the present invention may vary considerably, if present at all. However, they are generally present in amounts that are traditionally used for these added ingredients in known products in accordance with the present invention. Generally, the amount of additional ingredient(s) that can be present in the present invention is about 0.10 to 99.95 percent.

The cosmetic compositions of the present invention may and often do contain cosmetically acceptable coloring agents, e.g., pigments and dyestuffs, such as inorganic pigments, organic pigments, pearlescent pigments, and mixtures thereof. A pigment should be understood to mean inorganic or organic, white or colored particles.

Representative examples of inorganic pigments useful in the present invention include those selected from the group consisting of rutile or anatase titanium dioxide, coded in the Color Index under the reference CI 77,891; black, yellow, red and brown iron oxides, coded under references CI 77,499, 77, 492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

Representative examples of organic pigments and lakes useful in the present invention include, but are not limited to, D&C Red No. 19 (CI 45,170), D&C Red No. 9 (CI 15,585), D&C Red No. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No.3 (CI 45,430) and the dye or lakes based on Cochineal Carmine (CI 75,570) and mixtures thereof. A preferred organic pigment is carbon black.

Representative examples of pearlescent pigments useful in the present invention include those selected from the group consisting of the white or colored pearlescent pigments such as micalitanes, bismuth oxychloride, or titanium oxychloride.

Thickeners and gelling agents useful in accordance with the invention may include oil-phase thickeners or at least one agent useful for gelling a liquid fatty phase or mineral gelling agents. They also may include water soluble thickeners or gelling agents.

The thickening agents may be organic or inorganic compounds. Among organic compounds polymers are preferred. They can be associative or not associative polymers.

The thickening agent is generally present in an amount ranging from about 0.05% to about 20% by weight, and preferably from about 0.5% to about 10% by weight.

Plasticizers may be used which are organic compounds added to a high polymer both to facilitate processing and to increase the flexibility and toughness of the final product by internal modification of the polymer molecule. Examples of plasticizers include polyvinyl chloride, cellulose esters, phthalate esters, adipate esters, sebacate esters, ethylene glycol, tricresyl phosphate, and castor oil. Representative examples of plasticizers which may be included are glycol ethers, benzyl alcohol, triethyl citrate, 1,3-butylene glycol and propylene carbonate. The plasticizers generally are present in an amount from about 0.05% to about 40% by weight, and preferably from about 0.1% to about 40% by weight relative to the total weight of the composition. A preferred plasticizer is propylene carbonate.

The cosmetic compositions of the invention comprise a carrier that may be used for a cosmetic application (which is meant to include dermatologic and topical applications). The carrier may be any material that is cosmetically acceptable, and may include at least one organic solvent, such as a volatile solvent. The expression "volatile organic solvent" means an organic solvent that is capable of evaporating at room temperature from a support onto which it has been applied, in other words a solvent which has a measurable vapor pressure at room temperature. See, U.S. Patent 6,656,458. Representative examples of volatile organic solvents include volatile hydrocarbon-based oils. The expression "hydrocarbon-based oil" means an oil containing only hydrogen and carbon atoms. Examples of volatile hydrocarbon-based oils include isoparaffins, i.e., branched alkanes containing from 8 to 16 carbon atoms, and in particular isododecane (also known as 2,2,4,4,6-pentamethylheptane). It is also possible to use mixtures of such isoparaffins. Other volatile hydrocarbon-based oils, such as petroleum distillates, can also be used. Other useful organic solvents include C₁ - C₄ lower alkanols such as ethanol and isopropanol, glycerol, glycols and glycol ethers such as 2-butoxyethanol, propylene glycol, diethylene glycol monoethyl ether and monomethyl ether, and aromatic alcohols such as benzyl alcohol or phenoxyethanol, similar products and mixtures thereof. See, U.S. Patent 6,406,502. Yet other examples of organic solvents include volatile silicones such as, for example, cyclic and volatile silicone oils, such as cyclomethicone, octamethylcyclotetrasiloxane, decamethylcyclopentasiloxane, dodecamethylcyclohexasiloxane, linear volatile silicones such as octamethyltrisiloxane, heptamethylhexyltrisiloxane and heptamethyloctyltrisiloxane, or alternatively volatile fluoro oils such as nonafluoromethoxybutane or perfluoromethylcyclopentane.

The organic solvent(s) can be present in the composition according to the invention in a content ranging from about 1% to about 95% by weight, and preferably from about 5% to about 50% by weight. Nonorganic solvents such as water, may also be used.

The composition according to the invention may additionally comprise additional polymers such as film-forming polymers. The polymer can be selected from keratin derivatives, such as keratin hydrolysates and sulphonic keratins; anionic, cationic, amphoteric or nonionic derivatives of chitin or chitosan; cellulose derivatives such as hydroxyethylcellulose, hydropropylcellulose, methylcellulose, ethylhydroxyethylcellulose, carboxymethylcellulose, and quaternized derivatives of cellulose; acrylic polymers or copolymers, such as polyacrylates or polymethacrylates; polyvinylpyrrolidones (PVP) and vinyl copolymers, such as methyl vinyl ether-maleic anhydride copolymers, or vinyl acetate-crotonic acid copolymer; water-dispersible anionic polyesteramide and/or polyester polymers comprising monomers bearing a functional group -- SO₃M, in which M represents a hydrogen atom, an ammonium ion NH₄⁺ or a metal ion, such as, for example, an Na⁺, Li⁺, K⁺, Mg²⁺, Ca²⁺, Cu²⁺, Fe²⁺ or Fe³⁺ ion. Specific examples of the polymers described in U.S. Patents 3,734,874, 4,233,196 and 4,304,901. Polyurethane polymers, especially anionic, cationic, nonionic or amphoteric polyurethanes, acrylic polyurethanes, polyvinylpyrrolidone polyurethanes, polyester polyurethanes, polyether polyurethanes, polyureas, polyurea/polyurethanes, and mixtures thereof; and polymers of natural origin, modified if desired, such as gum arabic, guar gum, xanthan derivatives, karaya gum; alginates and carragheenates; glycoaminoglycans, hyaluronic acid and its derivatives; shellac, sandarac gum, dammars, elemis and copals, are also useful.

The polymer can be present in an amount ranging from about 0.05% to about 40% by weight, and preferably from about 0.1% to about 20% by weight.

Fillers that may be used in the compositions of the invention include, for example, silica powder; talc; polyamide particles and especially those sold under the name Orgasol by the company Atochem; polyethylene powders; microspheres based on acrylic copolymers, such as those based on ethylene glycol dimethacrylate/lauryl methacrylate copolymer sold by the company Dow Coming under the name Polytrap; expanded powders such as hollow microspheres and especially the microspheres sold under the name Expancel by the company Kemanord Plast or under the name Micropearl F 80 ED by the company Matsumoto; powders of natural organic materials such as crosslinked or noncrosslinked corn starch, wheat starch or rice starch, such as the powders of starch crosslinked with octenyl succinate anhydride, sold under the name Dry-Flo by the company National Starch; silicone resin microbeads such as those sold under the name Tospearl by the company Toshiba Silicone; clays (bentone, laponite, saponite, etc.) and mixtures thereof. These fillers may be present in amounts ranging from about 0.1% to about 40% by weight and preferably from about 0.5% to about 20% by weight relative to the total weight of the composition.

The sunscreens useful in the present invention typically comprise chemical absorbers, but may also comprise physical blockers. Exemplary sunscreens which may be formulated into the compositions of the present invention are chemical absorbers such as p-aminobenzoic acid derivatives, anthranilates, benzophenones, camphor derivatives, cinnamic derivatives, dibenzoyl methanes (such as avobenzone also known as Parsol^{®}1789), diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. These are variously described in U.S. Patents 2,463,264, 4,367,390, 5,166,355 and 5,237,071 and in EP 863,145, EP 517,104, EP 570,838, EP 796,851, EP 775,698, EP 878,469, EP 933,376, EP 893,119, EP 669,323, GB 2,303,549, DE 1,972,184 and WO 93/04665. Also exemplary of the sunscreens which may be formulated into the compositions of this invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

In its simplest form, products in accordance with the present invention include one or more MPPs in an amount ranging from a minimum of about 0.01 to about 60 percent by weight of the formulation in a carrier. The carrier can be a solvent, suspending agent, emulsion, colloid, or aerosol vehicle.

In another embodiment, the present invention relates to a process for imparting a visual or cosmetic effect onto a keratinous substrate comprising the steps of providing a cosmetic composition comprising a cosmetically acceptable carrier, multiphasic particles as described above, and optionally, at least one cosmetic additive for imparting an additional property to the keratinous substrate, and contacting the cosmetic composition with the keratinous substrate. The expression "providing a cosmetic composition" is meant to include all forms of obtaining or dispensing of the cosmetic composition prior to application by the user, whether performed by the user, or on behalf of the user. PREPARATION OF COSMETIC COMPOSITIONS

The following formulations comprise MPPs in various types of cosmetic compositions. All ingredient amounts are shown in weight percentage.

**Example 1 - Lip Gloss**

It is expected that the following lip gloss formulation will demonstrate improved properties compared to an identical formulation without the MPPs used in accordance with Example 1.

| | |
|---|---|
| Film Former (SA-70 from 3M) | 20.00% |
| Polyamidodimethylsiloxane | 8.00% |
| Phenyltrimethicone | 65.10% |
| MPP | 6.90% |

It is contemplated that the MPPs used in the formulation of Example 1 have two phases including a colorant encapsulated in one phase and an adhesive in the other phase. The MPPs in the formulation of Example 1 will have an average particle size of approximately 5 nm to 50 microns. The lip gloss formulation of Example 1 is expected to impart color to the lips.

The MPPs used in the formulation of Example 1 could also have three phases, including a colorant phase, an adhesive phase, and a phase comprising a material capable of imparting shine. A lip gloss formulation of Example 1 using these triphasic MPPs would be expected to impart color and shine to the lips.

**Example 2 - Foundation**

It is expected that the following foundation formulation will demonstrate improved properties compared to an identical formulation without the MPPs used in accordance with Example 2.

| | | |
|---|---|---|
| Phase A | Cyclopentasiloxane/Dimethicone copolyol | 8.00% |
| | Polyglyceryl-4 isostearate/Hexyl laurate/Cetyl PEG/PPG-10/1 Dimethicone | 3.50% |
| | Pigments | 9.90% |
| | | |
| Phase B | MPP | 6.00% |
| | | |
| Phase C | Preservative | 0.40% |
| | Disteardimethimonium Hectorite | 0.60% |
| | Propylene Carbonate | 0.20% |
| | | |
| Phase D | Magnesium Sulfate | 1.00% |
| | Preservative | 0.70% |
| | Non ionic emulsifier | 0.50% |
| | Water | q.s. to 100% |

It is contemplated that the MPPs used in the formulation of Example 2 have three phases including a colorant encapsulated in one phase, a UV filter encapsulated in a second phase, and a high skin substantive (adhesive) in a third phase. The MPPs in the formulation of Example 2 will have an average particle size of approximately 5 nm to 100 microns. The foundation formulation of Example 2 is expected to impart color to the skin and have sebum absorption properties.

**Example 3 - Sunscreen**

It is expected that the following sunscreen formulation will demonstrate improved properties compared to an identical formulation without the MPPs used in accordance with Example 3.

| Phase A | |
|---|---|
| Water | 31.40% |
| Avicel RC 591 (FMC Corp.) (microcrystalline cellulose (and) cellulose gum) | 1.50% |
| Rhodicare S 2% Solution (Rhodia) (xanthan gum) | 20.00% |
| Disodium EDTA | 0.10% |
| | |

| Phase B | |
|---|---|
| DC 200 Fluid 350 CS (Dow Corning) (dimethicone) | 6.00% |
| Cetyl Alcohol | 2.50% |
| Isostearate Isostearyle (Gattefossé) | 5.00% |
| Myritol 331 (Henkel) (cocoglycerides) | 4.00% |
| Labrafac CC (Gattefossé) (caprylic/capric triglycerides) | 8.50% |
| Parsol MCX (Roche) (octyl methoxycinnamate) | 6.00% |
| Parsol 1789 (Roche) (butyl dibenzoylmethane) | 1.50% |
| Eusolex 4360 (Merck) (benzophenone-3) | 1.50% |
| Vitamin E Acetate (Roche) (tocopheryl acetate) | 0.50% |
| | |

| Phase C | |
|---|---|
| MPP | 5.00% |
| | |

| Phase D | |
|---|---|
| Emulfree P (Gattefossé) (propylene glycol laurate (and) ethylcellulose (and) propylene glycol isostearate) | 6.00% |
| | |

| Phase E | |
|---|---|
| Gatuline A (Gattefossé) (pilewort extract) | 0.10% |
| Glydant Plus Liquid (Lonza) (DMDM hydantoin (and) iodopropynyl butylcarbamate) | 0.40% |

It is contemplated that the MPPs used in the formulation of Example 3 have three phases including a UV filter encapsulated in one phase, moisutrizer in a second phase, and an adhesive in a third phase. The MPPs in the formulation of Example 3 will have an average particle size of approximately 5 nm to 20 microns. The sunscreen formulation of Example 3 is expected to impart conventional sunscreen benefits, as well as a moisturizing effect.

**Example 4 - Mascara**

It is expected that the following mascara formulation will demonstrate improved properties compared to an identical formulation without the MPPs used in accordance with Example 4.

| | |
|---|---|
| Stearic acid | 5.82% |
| Beeswax | 3.07% |
| Carnauba wax | 3.21% |
| Paraffin | 12.76% |
| Preservative | 0.50% |
| Black Iron Oxide | 9.00% |
| Conditioning Agent | 6.13% |
| MPP | 3.25% |
| Panthenol | 0.50% |
| Preservative | 0.75% |
| Water | q.s. to 100% |

It is contemplated that the MPPs used in the formulation of Example 4 have two phases including a pigment encapsulated in one phase and a material that is substantive to the eyelash surface in the other phase. The MPPs in the formulation of Example 4 will have an average particle size of approximately 5 nm to 150 microns. The mascara formulation of Example 4 is expected to impart color to the eyelashes.

In the formulation of Example 4, the pigment phase could instead be a phase comprising a material capable of imparting lengthening properties to the eyelashes. This formulation would be expected to make the eyelashes longer. In addition, the MPPs of Example 4 could have all three phases - a pigment phases, a substantive to the eyelash surface, and a lengthening phases - resulting in a mascara formulation that would be able to impart color to and lengthen the eyelashes.

**Example 5 - Shampoo**

It is expected that the following shampoo formulation will demonstrate improved properties compared to an identical formulation without the MPPs used in accordance with Example 5.

| | |
|---|---|
| Lecithin | 0.10% |
| Polyquaternium-10 | 1.50% |
| Octylacrylamide/Acrylates/Butylaminoethyl methacrylate Copolymer | 1.50% |
| MPP | 1.50% |
| DMDM Hydantoin | 0.25% |
| Hexylene Glycol | 1.00% |
| Cocamidopropyl Betain | 5.00% |
| PPG-5-Ceteth-20 | 3.10% |
| Sodium Laureth Sulfate | 13.85% |
| Disodium Cocoamphodipropionate | 4.50% |
| Water | q.s. to 100% |

It is contemplated that the MPPs used in the formulation of Example 5 have two phases including a scalp care ingredient encapsulated in one phase and the other phase is a material that is substantive to the hair. The MPPs in the formulation of Example 5 will have an average particle size of approximately 5 nm to 60 microns. The shampoo formulation of Example 5 is expected to be soothing to the scalp.

**Example 6 - Conditioner (Leave On)**

It is expected that the following conditioner formulation will demonstrate improved properties compared to an identical formulation without the MPPs used in accordance with Example 6.

| | |
|---|---|
| Acrylamidopropyltrimonium chloride/Acrylamide Copolymer | 0.05% |
| PEG-45 Palm Kernel Glycerides | 0.50% |
| Glycereth-31 | 0.50% |
| Dimethicone Copolyol Meadowfoamate | 1.00% |
| DMDM Hydantoin | 0.50% |
| Polyquaternium-6 | 0.50% |
| PPG-5-Ceteth-20 | 1.00% |
| MPP | 2.00% |
| Fragrance | 0.25% |
| Phenoxyethanol | 0.50% |
| Water | q.s. to 100% |

It is contemplated that the MPPs used in the formulation of Example 6 have two phases including a material that is capable of imparting shine to the hair encapsulated in one phase and the other phase is a material that is substantive to the hair. The MPPs in the formulation of Example 6 will have an average particle size of approximately 5 nm to 60 microns. The conditioner formulation of Example 6 is expected to be impart shine to the hair.

The MPPs used in the formulation of Example 6 could also have three phases, including a shine phase, a substantive to the hair phase, and a UV filter phase. A conditioner formulation of Example 6 using these triphasic MPPs would be expected to have sunscreen properties and impart shine to the hair.

**Example 7 - Gel**

It is expected that the following gel formulation will demonstrate improved properties compared to an identical formulation without the MPPs used in accordance with Example 7.

| | |
|---|---|
| MPP | 5% |
| Acrylates/C-10-C30 Alkyl | 0.5% |
| Acrylate Crosspolymer PVP | 3.0% |
| Water | q.s. to 100% |

It is contemplated that the MPPs used in the formulation of Example 7 have two phases including an adhesive phase and a non-adhesive phase. The MPPs in the formulation of Example 7 will have an average particle size of approximately 5 nm to 60 microns. The gel formulation of Example 7 is expected to have hair styling properties.

**Example 8 - Emulsion**

It is expected that the following emulsion formulation will demonstrate improved properties compared to an identical formulation without the MPPs used in accordance with Example 8.

| | |
|---|---|
| MPP | 5% |
| Vaseline Oil | 20% |
| Acrylate Crosspolymer Carbomer | 0.3% |
| Water | q.s. to 100% |

It is contemplated that the MPPs used in the formulation of Example 8 have two phases including a hydrophilic phase and a hydrophobic phase. The MPPs in the formulation of Example 8 will have an average particle size of approximately 5 nm to 60 microns. The emulsion formulation of Example 8 is expected to impart a smooth feeling to the hair.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A cosmetic composition comprising:
(a) a cosmetically acceptable carrier;
(b) multiphasic particles having a surface and at least two homogenous phases which provide homogenous areas at said surface, each area imparting a different property or function, said multiphasic particles having an average particle size of about 1 nanometer to about 10 millimeters and said multiphasic particles present in an amount of between about 0.01 and about 60 percent by weight of said composition; and
(c) optionally, at least one cosmetic additive for imparting an additional property to a keratinous substrate.

2. The cosmetic composition of claim 1 wherein the at least two homogenous phases include at least one first phase which is hydrophobic and at least one second phase which is hydrophilic.

3. The cosmetic composition of claim 1 wherein the at least two homogenous phases include at least one first phase which is adhesive and at least one second phase which is non-adhesive.

4. The cosmetic composition of claim 1 wherein said multiphasic particles have an average particle size of about 100 nanometer to about 200 microns.

5. The cosmetic composition of claim 1 wherein said multiphasic particles include at least one phase which is polymeric.

6. The cosmetic composition of claim 1 wherein said multiphasic particles include at least one phase which is inorganic and/or metallic.

7. The cosmetic composition of claim 1 wherein said multiphasic particles are made of two hemispheres each of a different material.

8. The cosmetic composition of claim 1 wherein said multiphasic particles have a core and a coating, said core having a surface area, and said coating covering a portion of the surface area of the core.

9. The cosmetic composition of claim 1 wherein said cosmetic composition is foundation, blush, eye liner, a lip pencil, lipstick, nail polish, bronzing products, eye shadow, lip gloss, soap, deodorant, moisturizer, shampoo, conditioner, mousse, styling gel, styling spray, a depilatory, a hair coloring product, an exfoliant, baby powder, talcum powder, sunscreen, a sun tanning products, an antibacterial preparation, or an antifungal preparation.

10. The cosmetic composition of claim 1 wherein said cosmetically acceptable carrier is an organic solvent.

11. The cosmetic composition of claim 1 wherein said cosmetic additive is selected from the group consisting of thickening agents, plasticizers, coloring agents, antiwrinkle agents, astringents, skin-conditioning agents, sunscreens, and vitamins.

12. A process for imparting a visual or cosmetic effect onto a keratinous substrate comprising the steps of:
(a) providing a cosmetic composition comprising;
(i) a cosmetically acceptable carrier;
(ii) multiphasic particles having a surface and at least two homogenous phases which provide homogenous areas at said surface, each area imparting a different property or function, said multiphasic particles having an average particle size of about 1 nanometer to about 10 millimeters and said multiphasic particles present in an amount of between about 0.01 and about 60 percent by weight of said composition; and
(iii) optionally, at least one cosmetic additive for imparting an additional property to the keratinous substrate; and
(b) contacting the cosmetic composition with the keratinous substrate.

13. The process of claim 12 wherein at least one of the at least two homogenous phases provide at least an adhesive area, a nonadhesive area, a hydrophilic area, a hydrophobic area, a positive charge, a negative charge, a positive and negative charge, a porous area, a nonporous area, a rigid area, a flexible area, an area of bright finish, an area of matt finish, a transparent area, an opaque area, a colored area, a clear area, a diffractive area, a reflective area, an absorptive area, an antibiotic area, a UV-absorbing area, a UV-blocking area, a dense area, a smooth area, an irregular area, a textured area, a patterned area, a conductive area, a nonconductive area, a magnetic area, a metallic area, a reactive area, a nonreactive area, an area of generally good affinity for keratinaceous material and/or one that has no similar affinity, or any contradiction of these.

14. The process of claim 12 wherein said multiphasic particles have at least one phase which is hydrophobic, hydrophilic, adhesive, or nonadhesive.

15. The process of claim 12 wherein said multiphasic particles have an average particle size of about 100 nanometer to about 200 microns.

16. The process of claim 12 wherein said multiphasic particles are made of two hemispheres each of a different material.

17. The process of claim 12 wherein said multiphasic particles have a core and a coating, said core having a surface area, and said coating covering a portion of the surface area of the core.

18. The process of claim 12 wherein said multiphasic particles comprise at least three layers that form a multi-layered sheet.

19. The process of claim 12 wherein the at least one multiphasic particle has three or more phases.

20. The process of claim 12 wherein said multiphasic particles include at least one first phase which is hydrophobic and at least one second phase which is hydrophilic.

21. The process of claim 12 wherein said multiphasic particles include at least one first phase which is adhesive and at least one second phase which is non-adhesive.
